# EUROPÄISCHE PATENTANMELDUNG

(11) **EP 0 534 310 A1**
(43) Veröffentlichungstag der Anmeldung: **31.03.1993**
(21) Anmeldenummer: 92115887.9
(22) Anmeldetag: 17.09.1992
(51) Int. Cl.: A61K 47/36

(54) **Kombination enthaltend Wachstumsfaktoren und Polyelektrolyte**

(30) Priorität: 26.09.1991 DE 4132005
(71) Anmelder: MERCK PATENT GmbH, D-64271 Darmstadt (DE)
(72) Erfinder: Nies, Berthold, Dr., W-6101 Fränkisch-Crumbach (DE)

(57) **Zusammenfassung**

Die Erfindung betrifft Wirkstoffkombinationen, die aus mindestens einem Polypeptid mit der biologischen Wirkung von Fibroblasten-Wachstumsfaktoren und aus mindestens einem kationischen Polyelektrolyten bestehen, und die eine verbesserte Dosierung der FGF-Wirkung erlauben.

## Beschreibung

Die Erfindung betrifft Zusammensetzungen, die neben mindestens einem Polypeptid mit der biologischen Wirkung von Fibroblasten-Wachstumsfaktoren (FGF) mindestens einen kationischen Polyelektrolyten enthalten.

Fibroblasten-Wachstumsfaktoren (Fibroblast Growth Factors, FGF), die zur Klasse der körpereigenen Peptid-Wachstumsfaktoren gehören, wurden usprünglich als Substanzen in Gehirn und Hypophyse nachgewiesen und daraus isoliert und zeigten eine das Wachstum von Fibroblasten fördernde Aktivität. FGF's sind bekannt als wirksame gefäßbildende Faktoren, die u.a. für die Neovaskularisation bei der Wundheilung verantwortlich sind. Nähere Details zu FGF's einschließlich ihrer Abwandlungsprodukte, zu ihrer Isolierung bzw. Herstellung, ihrer Struktur, ihrer biologischen Aktivitäten und deren Mechanismen sowie zu entsprechenden medizinischen Anwendungen können der inzwischen umfangreichen Fachliteratur entnommen werden. Eine umfassende Übersicht bietet beispielsweise A. Baird and P. Böhlen, Fibroblast Growth Factors, in: Peptide Growth Factors and their Receptors I (editors: M.B. Sporn and A.B. Roberts) Springer Verlag Berlin, Heidelberg, New York 1990.

Als erfindungsgemäß geeignete Wachstumsfaktoren sind nicht nur die "klassischen" FGF's, wie der saure Fibroblasten-Wachstumsfaktor (acidic Fibroblast Growth Factor, aFGF) und der basische Fibroblasten-Wachstumsfaktor (basic Fibroblast Growth Factor, bFGF) anzusehen, sondern alle Peptide, die im wesentlichen die biologische Wirkung von FGF zeigen.

Zum engeren Kreis von FGFs zählen native FGF's, insbesondere bovinen und humanen Ursprungs, sowie rekombinant hergestellte FGF's. Bevorzugt sind insbesondere rekombinant hergestelltes humanes aFGF und bFGF. Näheres zu rekombinant hergestellten bovinen wie humanen aFGFs und bFGFs kann beispielsweise den folgenden Patentdokumenten entnommen werden: EP 0 228 449, EP 0 248 819, EP 0 259 953, EP 0 275 204. Zum weiteren Kreis von FGF's zählen auch Muteine, die sich von aFGF bzw. bFGF in einem gewissen Umfang in Zahl und/oder Sequenz der Aminosäuren unterscheiden, ohne daß hiermit eine wesentliche Wirkungsveränderung verbunden ist. Weiterhin sind unter dem Oberbegriff beispielsweise verschiedene Formen des bFGF's zu verstehen, die sich in ihrer Länge unterscheiden: Sie enthalten 146, 153, 154 beziehungsweise 157 Aminosäurereste. Der weitere Kreis von FGF's umfaßt schließlich noch verwandte Peptide mit zum Teil deutlich verschiedenen Aminosäuresequenzen, die aber die biologische Wirkung von FGF aufweisen. Als Literaturhinweis seien beispielhaft die folgenden Patentdokumente angeführt: EP 0 148 922, EP 0 226 181, EP 0 281 822, EP 0 288 307, EP 0 319 052, EP 0 326 907 und WO 89-12645. Die genannten Peptide werden zur Vereinfachung als "Peptide mit FGF-Wirkung" zusammengefaßt.

Zu FGF's im Sinne der Erfindung zählen weiterhin Derivate dieser Peptide, die mit stabilisierenden und/oder aktivitätssteigernden Agentien erhalten werden. Es sind dies insbesondere gegen Säure stabilisierte Formen von aFGF und bFGF, die als stabilisierende Agentien beispielsweise Glykosaminglykane wie Heparin, Heparinfragmente, Heparansulfat und Dermatansulfat oder Glukansulfate wie Dextransulfat und Cyclodextrinsulfat enthalten. FGF-Derivate dieser Art sind beispielsweise beschrieben in EP 251 806, EP 267 015, EP 312 208, EP 345 660, EP 406 856, EP 408 146, WO 89-12464, WO 90-01941 und WO 90-03797.

Bevorzugt für die vorliegende Erfindung sind FGF's humanen Ursprungs und deren Muteine, besonders bFGF. Besonders bevorzugt ist die Verwendung rekombinant hergestelltem humanen bFGF, wie in EP 0 248 819 beschrieben.

Gemeinsam ist den Peptiden mit FGF-Wirkung, daß sie sich spezifisch an die FGF-Rezeptoren der Zellmembran binden und dann ihre biologische Wirkung, z.B. bei der Wundheilung, entfalten. Jedoch werden diese Peptide auch unspezifisch gebunden. Diese Bindung führt dazu, daß z.B. bFGF selbst nach Injektion üblicherweise nicht im Serum nachgewiesen werden kann. Beispielsweise wird bFGF als basisches Protein (IP = 9,8) an anionische Makromoleküle (z. B. Nukleinsäuren und saure Komponenten der extrazellulären Matrix (ECM) wie Heparansulfat - s. u.a. Moscatelli et al.: Interaction of basic fibroblast growth factor with extracellular matrix and receptors. Vortrag anläßlich einer Konferenz zu "The Fibroblast Growth Factor Family" veranstaltet von The New York Academy of Sciences, 1991) gebunden. Diese Eigenschaft ist in der Literatur beschrieben und wird u.a. dazu ausgenutzt FGF's zu isolieren: Reinigung von a- und bFGF mittels Chromatographie auf Trägern, die Heparin gebunden enthalten.

Die unspezifische Bindung der Peptide mit FGF-Wirkung verringert die FGF-Menge, die den spezifischen Rezeptor auf der Zelle erreichen kann. Insbesondere bei älteren (chronischen) Wunden oder Verbrennungswunden, oder wenn große Mengen nekrotischen Materials vorliegen, wird die unspezifische Bindung einen großen und zugleich unbekannten Teil des FGF absorbieren. Deswegen ist eine genaue Dosierung der wirksamen Menge an Peptiden mit FGF-Wirkung nicht möglich. Es besteht die Notwendigkeit zur Überdosierung des Wirkstoffs. Damit ist die Dosierung auch unzuverlässig, da unbekannt ist, wieviele unspezifische Bindungsstellen in der jeweiligen Wunde vorhanden sind.

Versuche bFGF zu stabilisieren, seine unspezifische Bindung zu verringern und seine Affinität zum spezifischen Rezeptor zu erhöhen, bestanden bisher darin, bFGF mit Heparin oder anderen sulfatierten Glycanen (z.B. Dextransulfat) oder Sucralfat zu kombinieren. Üblicherweise wird bFGF z.Z. jedoch ohne solche Zusätze getestet. Es werden lediglich Hilfsstoffe zugesetzt, die der Haltbarkeit und verbesserten Appliziebarkeit dienen.

Der Nachteil bei der Verwendung von reinem bFGF und in unterschiedlich starkem Maße auch bei den genannten Kombinationen und Derivaten besteht in der Notwendigheit zur Überdosierung des Wirkstoffs und der Unzuverlässigkeit der Dosierung, da unbekannt ist, wieviele unspezifische Bindungsstellen in der jeweiligen Wunde vorhanden sind.

Der Erfindung lag daher die Problemstellung zugrunde, eine Wirkstoffkombination zur Verfügung zu stellen, deren biologische Aktivität von der unspezifischen Bindung der Peptide mit FGF-Wirkung nicht beeinflußt ist.

Für eine verbesserte Dosierung mittels Diffusionskontrolle wird zwar in EP 0 312 208 vorgeschlagen, Gelbildner zusammen mit Wachstumsfaktoren, bevorzugt Epidermalem Wachstumsfaktor (EGF), einzusetzen. Jedoch wird durch diese Maßnahme das grundlegende Problem bei der Dosierung von Wachstumsfaktoren, wie es aus der variablen unspezifischen Bindung resultiert, nicht gelöst.

Es wurde nun gefunden, daß diese Dosierungsprobleme gelöst werden können, wenn man bei der Applikation von Peptiden mit FGF-Wirkung zusätzlich mindestens einen kationischen Polyelektrolyten appliziert. Überraschend ist dabei, daß die kationischen Polyelektrolyte zwar die unspezifischen Bindungsstellen, z.B. in der EGM, blockieren, daß aber die Peptide mit FGF-Wirkung dennoch ihre spezifischen Rezeptoren in der Zellmembran erreichen können, und daß somit die biologische Wirkung des FGF erhalten bleibt.

Eine Anzahl von bekannten kationischen Polyelektrolyten läßt sich erfindungsgemäß benutzen. Gemeinsam ist ihnen, daß sie unter physiologischen Bedingungen mit unspezifisch FGF-bindenden Zellstrukturen irreversibel Komplexe bilden. Beispiele für geeignete kationische Polyelektrolyte sind Polyamine und -imine, sowie deren quaternisierte Derivate. Weiterhin sind Polyguanidine geeignet, sowie Polypeptide, die basische Aminosäuren enthalten; z.B. Polylysin, Polyornithin, Polyarginin. Auch können derartige Polypeptide Gamische von mehreren Aminosäuren enthalten. Die Aminosäuren selbst können entweder racemisch oder als einheitliche D- oder L-Form eingesetzt in den erfindungsgemäß benutzten Peptiden vorkommen. Geeignet sind weiterhin Polysaccharide mit basischen Gruppierungen. Besonders bevorzugt ist die Verwendung von Chitosanen und ihren Derivaten, z.B. N-Carboxy-butyl-chitosan oder Methyl-Pyrrolidon-Chitosan, da diese biodegradierbar sind und die Abbauprodukte mit Komponenten des normalen Stoffwechsels (Glucosamin und N-Acetyl-Glucosamin) identisch sind.

Erfindungsgemäß können die kationischen Polyelektrolyte als freie Basen oder auch als Salze mit physiologisch verträglichen organischen oder anorganischen Säuren eingesetzt werden. Physiologisch verträgliche organische Säuren sind beispielsweise Glutaminsäure, Essigsäure und Milchsäure. Salzsäure ist als Beispiel für eine physiologisch verträgliche anorganische Säure genannt. Weitere physiologisch verträgliche Säuren sind dem Galeniker bekannt.

Diese Substanzen werden im Überschuß zu den Peptiden mit FGF-Wirkung gleichzeitig mit diesen appliziert. Ebenso ist eine vorherige Applikation der kationischen Polyelektrolyte möglich. Dadurch können FGF's ungehindert bis zum spezifischen Rezeptor diffundieren und an diesen binden. Es ist aber auch im Rahmen des erfindungsgemäßen Vorgehens denkbar, die kationischen Polyelektrolyte nachträglich zu applizieren, um die FGF's von den unspezifischen Bindungsorten zu verdrängen. Der Vorteil dieser Vorgehensweise ist in jedem Fall, die Peptide mit FGF-Wirkung vergleichsweise niedrig dosieren zu können, wodurch der therapeutische Index entsprechend erhöht wild. Zudem ist eine wesentlich zuverlässigere Anwendung ereichbar, da selbst bei stark nekrotisierenden Wunden die FGFs' an den Zielort gelangen können. In derartigen Fällen ist eine mehrfache Applikation häufig indiziert.

Gegenstand der Erfindung sind daher Kombinationen, die mindestens ein Polypeptid mit FGF-Wirkung und mindestens einen kationischen Polyelektrolyten enthalten.

Die erfindungsgemäßen Kombinationen können dabei als bevorzugt wäßrige Lösung en vorliegen die neben mindestens einem Polypeptid mit FGF-Wirkung und mindestens einem kationischen Polyelektrolyten übliche Zusätze, wie Puffersubstanzen oder Salze zur Regulierung des osmotischen Druckes oder auch Füllstoffe, enthalten. In diesen Lösungen liegt die Konzentration der Polypeptide mit FGF-Wirkung zwischen 0,1 ng/ml und 500 µg/ml, vorzugsweise zwischen 0,5 ng/ml und 300 µg/ml, die der kationischen Polyelektrolyten zwischen 1 µg/ml und 300 mg/ml, vorzugsweise zwischen 0,1 und 200 mg/ml. Diese Lösungen können in bekannter Weise durch Zusatz von Gelbildnern zu Hydrogelen weiter verarbeitet werden. Die Lösungen, gegebenenfalls unter Zusatz von Füllstoffen, oder Hydrogele können zu Vliesmaterialien, zu Folien, zu Pulvern, zu Granulaten oder zu Fäden getrocknet oder lyophilisiert werden. Solcherart erhaltene Fäden können zu Geweben oder Netzen weiterverarbeitet werden. Auch ist es möglich, diese Lösungen in Wundgaze oder in Wundverschlußfolie zu absorbieren und diese zu trocknen. Die getrockneten Darreichungsformen können auch mit Flüssigkeit rekonstituiert als wäßrige Lösung oder als Gel oder Hydrogel zur wundversorgung benutzt werden. Die erfindungsgemäßen Kombinationen können schließlich auch als Reagenzzusammenstellung (Kit) vorliegen, bei dem die Komponenten (Peptid mit FGF-Wirkung und kationischer Polyelektrolyt) getrennt, aber in abgestimmten Mengen vorliegen.

Bei frischen sauberen Wunden ist ein geringer Überschuß an kationischen Polyelektrolyt, d.h. zumindest etwa das fünffache der Menge an Peptiden mit FGF-Wirkung, für die Maskierung ausreichend. Entsprechend dem Wundzustand muß dieser Überschuß aber erheblich erhöht werden, um die unspezifischen Bindungsstellen zu blockieren. Deswegen kann erfindungsgemäß die Menge an kationischem Polyelektrolyten bis zum Zehntausendfachen der Menge des Peptides mit FGF-Wirkung betragen. Bevorzugt beträgt das Verhältnis zwischen den Gewichtsmengen an kationischen Polyelektrolyt und Peptid mit FGF-Wirkung zwischen 100 und 2000.

Gegenstand der Erfindung ist weiterhin die Verwendung eines Zusatzes von mindestens einem kationischen Polyelektrolyten zu einer Präparation, die mindestens ein Peptid mit FGF-Wirkung enthält, um unspezifische Bindungsstellen für das Peptid mit FGF-Wirkung zu blockieren.

Gegenstand der Erfindung sind Verfahren zur Herstellung von Kombinationen, die mindestens ein Peptid mit FGF-Wirkung und zusätzlich mindestens einen kationischen Polyelektrolyten enthalten, wobei die Komponenten bevorzugt als wäßrige Lösung vorliegen, und weiterhin übliche Zusätze, wie Puffersubstanzen oder Salze zur Regulierung des osmotischen Druckes oder auch Füllstoffe enthalten können. Diese Lösungen können auch sterilisiert werden. Es ist außerdem möglich, diese Lösungen zu halbfesten oder festen Darreichungsformen, z.B. Hydrogelen, Vliesen, Fäden, Geweben, Granulaten, Pulvern, getränkten Materialien weiterzuverarbeiten.

Gegenstand der Erfindung sind Verfahren zur Herstellung pharmazeutischer Zubereitungen, bei denen eine Kombination, die mindestens ein Peptid mit FGF-Wirkung und zusätzlich mindestens einen kationischen Polyelektrolyten enthält, mit mindestens einem festen, flüssigen oder halbflüssigen Träger- oder Hilfsstoff in eine geeignete Dosierungsform gebracht wird.

Gegenstand der Erfindung sind pharmazeutische Zubereitungen, die Kombinationen, bestehend aus mindestens einem Peptid mit FGF-Wirkung und zusätzlich mindestens einem kationischen Polyelektrolyten, enthalten.

Gegenstand der Erfindung ist die Verwendung von Kombinationen, die mindestens ein Peptid mit FGF-Wirkung und zusätzlich mindestens einen kationischen Polyelektrolyten enthalten, zur Bekämpfung von Krankheiten.

Gegenstand der Erfindung ist die Verwendung einer Kombination die mindestens ein Peptid mit FGF-Wirkung und zusätzlich mindestens einen kationischen Polyelektrolyten enthält, zur Herstellung eines Arzneimittels.

Die erfindungsgemäßen Kombinationen können vorteilhaft bei der Therapie von Wunden, für die eine Indikation einer FGF-Therapie besteht, eingesetzt werden, besonders wenn bei der Heilung dieser Wunden oder Defekte Vorgänge der freien Granulation involviert sind. Wichtige Anwendungsgebiete für die erfindungsgemäßen Kombinationen sind Ulcera der Haut (beispielsweise Dekubitalulcera, diabetische Gangräne, arterielle und venöse Stauungen), weiterhin Verbrennungswunden, Schleimhautulcera und -läsionen, Spenderhautentnahmestellen, Haut- und Weichteiltransplantationsstellen (Vorbereitung des Wundgrundes) und Operationswunden (insbesondere bei eingeschränkten Heilungsfunktionen). Für alle diese Anwendungsgebiete ist die Applikation von Kombinationen aus Peptiden mit FGF-Wirkung und geeigneten Polykationen besonders dann wichtig, wenn es sich um Patienten mit eingeschränkter Wundheilungsfähigkeit handelt (z.B. aufgrund von Alter, Grundkrankheiten oder therapeutischen Maßnahmen).

### Beispiele

Die Herstellung und Verwendung der erfindungsgemäßen Kombinationen wird im folgenden beispielhaft beschrieben, ohne daß in dieser Beschreibung eine Einengung des Erfindungsgegenstandes zu sehen ist. Die Vorteile gegenüber den bislang aus dem Stand der Technik bekannten FGF-haltigen Präparationen werden durch die mitgeteilten Anwendungsbeispiele deutlich.

### Beispiel 1

### Beispiel 1a: Chitosan-Lösung

100 mg Chitosan Glutamat (Protan) werden in 10 ml Ringerlösung gelöst.

### Beispiel 1b: bFGF-Lösung

100 µg rekombinant hergestelltes humanes bFGF werden in 10 ml 20 mM Citrat-Puffer pH 5,0 gelöst.

### Beispiel 1c: kombinierte Chitosan-bFGF-Lösung

Je 10 ml der Lösungen nach Beispiel 1a und 1b werden gemischt. Die Endkonzentration an bFGF beträgt 5 µg/ml.

### Beispiel 2

### Beispiel 2a: Chitosan-Lösung

100 mg Methyl-Pyrrolidon-Chitosan werden in 10 ml wäßriger Kochsalzlösung (9 g/l) gelöst.

### Beispiel 2b: bFGF-Lösung

50 µg rekombinant hergestelltes humanes bFGF werden in 10 ml 20 mM Citrat-Puffer pH 5,0 gelöst.

### Beispiel 2c: kombinierte Chitosan-bFGF-Lösung

Je 10 ml der Lösungen nach Beispiel 2a und 2b werden gemischt. Die Endkonzentration an bFGF beträgt 2,5 µg/ml.

### Beispiel 3

Die Lösung aus Beispiel 1c wird nach Herstellung in 2-5 mm Schichtdicke lyophilisiert. Es entsteht ein wirkstoffhaltiges Vlies, das zur Wundabdeckung geeignet ist.

### Beispiel 4

Handelsübliche Wundverbandgaze wird mit der Lösung aus Versuch 2c getränkt ( 2 ml/5 cm²) und anschließend getrocknet. Es entsteht eine wirkstoffhaltige Gaze, die zur Wundabdeckung geeignet ist.

### Anwendungsbeispiel A

Die visköse Lösung aus Beispiel 1c wird gleichmäßig auf die zu behandelnde Wunde mit ca. 1 ml/ 5 cm² wundfläche aufgetragen.

### Anwendungsbeispiel B

Zunächst wird die Wunde mit einer Lösung nach Beispiel 1a (Chitosan) vorbehandelt (ca. 1 ml / 5 cm² Wundfläche). Nach einer Vorbehandlungszeit von ca. 30 Minuten wird die überschüssige Lösung und Wundsekret abgetupft. Anschließend wird die Wunde mit einer Lösung nach Beispiel 1b (bFGF) weiterbehandelt (ca. 1 ml / 5 cm² Wundfläche).

### Anwendungsbeispiel C

Zunächst wird die Wunde mit einer Lösung nach Beispiel 1a (Chitosan) vorbehandelt (ca. 1 ml / 5 cm² Wundfläche). Nach einer Vorbehandlungszeit von ca. 30 Minuten wird die überschüssige Lösung und Wundsekret abgetupft. Anschließend wird die Wunde mit einer Lösung nach Beispiel 1c (Kombination aus Chitosan und bFGF) weiterbehandelt (ca. 1 ml / 5 cm² Wundfläche).

Diese Methode hat den Vorteil, daß durch das Auftragen der Kombination aus bFGF und Chitosan neu ins Wundgebiet sekretierte bFGF-bindende Substanzen absorbiert werden.

## Patentansprüche

1. Kombination enthaltend mindestens ein Peptid mit FGF-Wirkung und zusätzlich mindestens einen kationischen Polyelektrolyten.

2. Verfahren zur Herstellung einer Kombination nach Anspruch 1, dadurch gekennzeichtnet, daß eine wasserhaltige Lösung mindestens eines Peptides mit FGF-Wirkung mit einer wasserhaltigen Lösung mindestens eines kationischen Polyelektrolyten zusammengebracht werden.

3. Verfahren zur Herstellung einer pharmazeutischen Zubereitung, dadurch gekennzeichnet, daß man eine Kombination nach Anspruch 1 mit mindestens einem festen, flüssigen oder halbflüssigen Träger- oder Hilfsstoff in eine geeignete Dosierungsform bringt.

4. Pharmazeutische Zubereitung, dadurch gekennzeichnet, daß sie mindestens eine Kombination nach Anspruch 1 enthält.

5. Kombination nach Anspruch 1 zur Bekämpfung von Krankheiten.

6. Verwendung einer Kombination nach Anspruch 1 zur Herstellung eines Arzneimittels.

7. Verwendung einer Kombination nach Anspruch 1 bei der Bekämpfung von Krankheiten.

8. Verwendung nach Anspruch 7, daurch gekennzeichnet, daß man zunächst eine Komponente, enthaltend den kationischen Polyelektrolyten, und anschließend eine zweite Komponente, die das Peptid mit FGF-Wirkung enthält, einwirken läßt.
